(19) Europäisches Patentamt
European Patent Office
Office européen des brevets

(11) **EP 2 295 964 A1**

(12) **EUROPEAN PATENT APPLICATION**

(43) Date of publication:
**16.03.2011 Bulletin 2011/11**

(51) Int Cl.:
***G01N 33/50*** (2006.01)    ***A61K 31/19*** (2006.01)

(21) Application number: **09011667.4**

(22) Date of filing: **11.09.2009**

| | |
|---|---|
| (84) Designated Contracting States:<br>**AT BE BG CH CY CZ DE DK EE ES FI FR GB GR HR HU IE IS IT LI LT LU LV MC MK MT NL NO PL PT RO SE SI SK SM TR**<br>Designated Extension States:<br>**AL BA RS**<br><br>(71) Applicant: **Fresenius Kabi Deutschland GmbH**<br>**61352 Bad Homburg v.d.H. (DE)** | (72) Inventor: **Hahn, Robert G., Prof. Dr.**<br>**14640 Tullinge (SE)**<br><br>(74) Representative: **Hollatz, Christian**<br>**ter Meer Steinmeister & Partner GbR**<br>**Mauerkircherstrasse 45**<br>**D-81679 München (DE)** |

(54) **Irrigation solution**

(57)    The invention generally relates to methods for delivering information on fluid absorption. More particularly, the invention relates to methods to monitor the fluid absorption of a human or animal body and to methods for obtaining a set of data of a human or animal body. The invention also relates to surgical irrigation fluid compositions and ready-to-use kits, which can be used in the methods.

Fig. 1

EP 2 295 964 A1

**Description**

**Technical field**

The invention generally relates to methods for delivering information on fluid absorption. More particularly, the invention relates to methods for monitoring and obtaining a set of data on the fluid absorption of a human or animal body. The invention also relates to surgical irrigation fluid compositions and ready-to-use kits, which can be used in the above methods.

**Background**

During endoscopic procedures of the genitourinary tract rinsing procedures of the operation vicinity are employed. However, conventional rinsing processes entail the risk of irrigation fluid absorption to the body. Absorption in excess of 3 L of electrolyte-free fluid causes symptoms, which are generally summarized as the transurethral resection (TUR) syndrome, which is a rare but potentially life-threatening complication. Transurethral resection of the prostate is called TURP. Early detection of fluid absorption during endoscopic procedures is crucial for evaluating the risk of developing syndromes, such as the TUR syndrome. Moreover, when indicated, counter measures can be applied at an early stage, thus mitigating the symptoms and ultimately saving lives.

One established method for detecting the absorption of fluid to the human body is to measure the exhaled breath concentration of either ethanol or nitrous oxide that is added as a tracer substance to the irrigating solution. In another method, the fluid output and input is measured or the patient is weighed on-table. All these methods, however, must be applied with great care to be reasonably accurate. In addition to these methods being cumbersome or unfamiliar, the relatively low frequency of massive fluid absorption occurrences explains why fluid absorption is generally not monitored continuously during surgery.

Furthermore, while the postoperative serum sodium concentration can be assessed to confirm or refute fluid absorption during monopolar electrocautery, this is not possible when using bipolar electrocautery, because this method typically uses 0.9% NaCl for irrigation and therefore, the fluid absorption can no longer be confirmed by a decreased serum sodium concentration.

Moreover, the detection of exhaled ethanol is slow, small amounts of ethanol cannot be detected with a high accuracy and other side effects with respect to the patient occur, such as dizziness or even alcohol dependence.

**Technical object and solution**

In view of the above problems generally observed in the prior art, it was the technical object of the present invention to provide methods for the delivery of information on fluid absorption in a human or animal body, which do not have the above disadvantages and which can be used in combination with endoscopic procedures including both, monopolar and bipolar electrocautery. Another object of the invention is to provide surgical irrigation fluids and ready-to-use kits employable in the methods.

The above technical object is solved by the methods, surgical irrigation fluids, ready-to-use kits and practical uses as described in the claims. In particular, it has surprisingly been found by the inventors of the present invention that carbohydrates, such as glucose, can be used as a marker for obtaining accurate and reproducible information on the fluid absorption by a human or animal body. The distribution kinetics of the carbohydrate compounds makes it possible to measure the concentration thereof in the body fluids from the patient during surgery of the kind disclosed herein. Furthermore, the concentration of the carbohydrate compound in the body fluid is essentially proportional to the absorbed amount of irrigation fluid. In addition hereto, the use of carbohydrate compounds shows great advantages as compared to the use of the state of the art tracer substances, such as ethanol, nitrous oxide and serum sodium. Some of these advantages are described the following.

When a carbohydrate compound is added as a marker, even small amounts of the absorbed rinsing or irrigation fluid can be detected within a relatively short time, because the quantification of carbohydrate compounds, for example the serum glucose concentration, in body fluids is a routine analytical process in clinical laboratories. In other words, the present invention enables the rapid and accurate detection of irrigation fluid absorbed by an animal or human body. In consequence, necessary countermeasures can be taken at an early stage and in fact even before symptoms occur. The carbohydrate compounds can be added to the irrigation fluid in advance and stored for several years, if kept sterile under appropriate conditions. In other words, a ready-to-use kit can be obtained, which comprises the irrigation fluid.

These methods can be used in combination with both, monopolar or bipolar electrocautery in endoscopic procedures, for example monopolar and bipolar TUR surgery or TURP surgery, with high accuracy as well as with excellent reproducibility in all cases. The data can easily be obtained by laboratory and/or bedside measurement in the same way as for the state of the art serum sodium quantification, if an irrigation fluid absorption is suspected. Undesired side effects, such as dizziness and even alcohol dependence, are not observed.

**Detailed description**

In one aspect, there is disclosed a method to monitor the fluid absorption of a human or animal body. The method comprises the following steps: (a) contacting a human or animal body with a fluid composition comprising at least one marker compound, (b) taking a body fluid sample from the human or animal body, (c) measuring the concentration of the marker compound or one of its metabolites in the body fluid sample, and (d) calculating the fluid absorption of the body from the concentration of the marker compound or one of its metabolites in the body fluid sample. Preferably, the marker compound is a physiologically acceptable carbohydrate compound.

The term fluid, as used herein, generally refers to any substance capable of flowing and easily changing shape. For example, the fluid can be a liquid substance, which may comprise one or more solutes or partially or totally dissolved substances.

The term body fluid, as used herein, refers to any fluid that is excreted or secreted by the body as well as body liquids that normally are not. For example, the body fluid may be one or more fluids selected from the group consisting of blood, blood plasma (serum), saliva, tears, urine, amniotic fluid, aqueous humour, cerumen, chime, interstitial fluid, lymph, breast milk, mucus, sebum, semen, sweat and vomit. Preferably, the body fluid is blood or blood plasma, which can be obtained with ease from the patient by venipuncture or other methods known in the art.

The term carbohydrate compound, as used herein, refers to any carbohydrate, which can be used to deliver biological or physiological data from a human or animal body or that can be used to trace and/or quantify the fluid absorption of a human or animal body by measuring its concentration, as long as it is physiologically acceptable. Preferably, the carbohydrate compound is soluble in water and/or is soluble in at least one body fluid.

In general, the carbohydrate compound can be a monosaccharide, a disaccharide or an oligosaccharide. In a preferred embodiment, the carbohydrate compound is a monosaccharide. The carbohydrate compound may be an aldose, if it contains at least one aldehyde functionality, or may be a ketose, if it contains at least one keto functionality.

If the carbohydrate is a monosaccharide, it may be in the form of an aldotriose, an aldotetrose, an aldopentose or an aldohexose or may be in the form of a ketotriose, a ketotetrose, a ketopentose or a ketohexose. Examples for monosaccharides that can be used according to this disclosure are D-glyceraldehyde, D-erythrose, D-threose, D-ribose, D-arabinose, D-xylose, D-lyxose, D-allose, D-altrose, D-glucose, D-mannose, D-gulose, D-idose, D-galactose or D-talose. If the monosaccharide is in the form of a ketose, it may be selected from the group consisting of dihydroxyacetone, D-erythrulose, D-ribulose, D-xylulose, D-psicose, D-fructose, D-sorbose or D-tagatose.

Preferably, the carbohydrate is D-glucose (generally referred to as glucose throughout this disclosure), which provides for an excellent physiological acceptance and can easily be quantified in the laboratory by a standard serum glucose assay. Moreover, if the carbohydrate is D-glucose, the fluid absorption of a human or animal body can be monitored and data can be obtained in a particularly accurate and reproducible manner. In a preferred implementation, the marker compound is a mixture of one or more of the carbohydrate compounds as described above.

The term metabolite, as used herein, refers to any product, which can directly or indirectly be produced from the marker compound by metabolism in a human or animal body. Preferably, the concentration of the marker compound or one of its metabolites in the body fluid sample is measured in a laboratory.

An alternatively preferred aspect of the invention involves the use of a partially or completely ionized or solubilized metal cation or counter-anion to measure the amount of irrigation fluid absorption as a marker substance, the marker being preferably sodium. This marker can gainfully be employed in combination with one of the above described markers.

In a further aspect, there is disclosed a method for obtaining a set of data of a human or animal body. The method comprises the following steps: (a) bringing into contact a human or animal body with a fluid composition comprising at least one marker compound, (b) taking a body fluid sample from the human or animal body, (c) measuring the concentration of the marker compound or one of its metabolites in the body fluid sample, (d) compiling a set of data from the concentration of the marker compound or one of its metabolites in the body fluid sample. Preferably, the marker compound is a physiologically acceptable carbohydrate compound.

The terms fluid, body fluid, carbohydrate compound and metabolite are as described above.

Preferably the concentration measurement and data compilation steps occur in a laboratory.

The term data, as used herein, refers to any piece of information that represents the qualitative or quantitative attribute of a variable or set of variables and which can be compiled from the concentration of the marker compound in the body fluid. Preferably, the set of data is the amount of irrigation fluid absorbed in a human or animal body during surgery. In this case, the amount of irrigation fluid absorbed in a human or animal body during surgery may be calculated by multiplying the concentration of the marker compound in the body fluid by a corrective factor. Typically, the corrective factor will be a constant dependent on the type of patient. In particular, the corrective factor will depend on the gender, age and medical conditions of the patient and will be different if the patient has developed diabetes. For example, for healthy male patients aged 20 to 41 years and having a body weight of 72 to 111 kg, the corrective factor will be approximately 0.27 $L^2$/mmol. In other words 1 L of fluid absorption corresponds to a serum carbohydrate compound concentration increase (e.g. serum glucose concentration increase) of 3.7 mmol/L.

In a preferred implementation in step (d) the fluid absorption is calculated or the set of data is compiled as a function of time.

In a further aspect, the steps (b) and (c) are carried out at least twice.

Without wishing to be bound by theory, it has been observed that if the set of data is compiled as a function of time, the accuracy of the data obtained can be further increased. Moreover, since the marker compound is decomposed in the human or animal body following a predeterminable clearance curve known to the skilled person, the body fluid sample may be taken from the human or animal body even after a surgery has been completed. In this case, the fluid absorption can still be calculated from the marker compound concentrations by evaluating the amount of the marker compound in the body and the time interval between surgery and sampling of the body fluid.

In a further implementation, the methods as described above further comprise a step (e): assigning the set of data obtained in step (d) to a medicinal condition. For example, if the set of data is the fluid absorption of a human or animal body during endoscopic surgery, the medical condition is preferably a prognosis, whether or not the animal or human patient will develop a TUR syndrome.

In yet a further aspect, there is disclosed a surgical irrigation fluid composition comprising a rinsing fluid and a marker compound. The rinsing fluid comprises sterile water and the marker substance is preferably a physiologically acceptable carbohydrate compound in an amount detectable via a human or animal body, which is subject to surgery. The marker compound is as described above.

Preferably, the marker compound is completely dissolved in the rinsing fluid. In this case the surgical irrigation fluid composition can be stored and applied as a solution, wherein no clogging or blockage of the application tool is observed upon use and the concentration of the marker compound in the solution does not change upon storage. If the marker compound is a carbohydrate compound, the irrigation fluid composition is a clear solution, which is particularly preferable, because visual inspections and photography accompanying the endoscopic surgery will not be impeded by any turbidity of the fluid at the location of the operative procedure.

In order to obtain the surgical irrigation fluid composition, the marker compound is simply mixed with the rinsing fluid, wherein the mixing is preferable carried out until the marker compound is completely dissolved in the rinsing fluid, thereby obtaining a clear solution.

The term surgical irrigation fluid composition, as used herein, refers to any fluid that is suitable for irrigating at least one part of a human or animal body during a surgical procedure, for example an endoscopic surgery. The irrigation fluid composition thereby enables the surgeon to gently dilate mucosal spaces and to remove blood and cut tissue from the operating field. The term sterile water, as used herein, refers to any water that does not contain any active bacterial, fungal or viral contaminants. Sterility of the water is necessary to avoid contamination or infection of the human or animal body during the surgical procedure after irrigation with the surgical irrigation fluid composition. Sterility can be obtained by methods known in the art, such steam autoclaving or sterile filtration.

The rinsing fluid referred to herein is selected in accordance with knowledge known in the art.

In a preferred implementation, the physiologically acceptable carbohydrate compound is glucose. Preferably, the surgical irrigation fluid composition comprises glucose in such an amount that the concentration in the fluid sample subjected to the surgery in question is detectable by a glucose detector known in the art. However, preferably the concentration of glucose in the surgical irrigation fluid composition is from 1 to 10 wt.-%, more preferably from 2.5 to 7.5 wt.-%, and even more preferably 5 wt.-%, based on the weight of the rinsing fluid. In one implementation, the fluid composition contains 95 wt.-% sterile water and 5 wt.-% glucose, based on the weight of the rinsing fluid.

In a preferred implementation, the surgical irrigation fluid composition further comprises a haemolysis preventing solute and/or a viscosity enhancing solute as an additive. The haemolysis preventing solute is generally selected in accordance with general knowledge within this field. A preferred haemolysis preventing solute is selected from the group consisting of glycine, mannitol, sorbitol and mixtures thereof.

If present in the surgical irrigation fluid composition, the solute is preferably utilized within the range of 0.1 to 5.0 wt.-%, more preferably 0.5 to 2.5 wt.-%, even more preferably 1.0 to 2.0 wt.-%, based on the weight of the rinsing fluid. It is preferred that also the haemolysis preventing solute and the viscosity enhancing solute is completely dissolved in the surgical irrigation fluid, thereby providing a clear solution.

In a preferred implementation, the rinsing fluid comprises Ringer's acetate solution. Ringer's acetate solution is isotonic with blood and optimized for intravenous administration. One liter of Ringer's acetate solution contains the following ions: 130 mEq of sodium ion, 109 mEq of chloride ion, 28 mEq of acetate, 4 mEq of potassium ion, 3 mEq of calcium ion. For example, the sodium, chloride, potassium and acetate originate from sodium chloride (NaCl), Sodium acetate (NaAc), calcium chloride ($CaCl_2$) and potassium chloride (KCl). Ringer's acetate solution is commercially available.

In some implementations, the surgical irrigation fluid composition further comprises additional additives or excipients known in the fields. For example, if the surgical procedure comprises at least one bipolar electrosurgery step, the surgical irrigation fluid composition may further comprise 1 to 20 wt.-% sodium chloride, or 4 to 12 wt.-% sodium chloride, more preferably 9 wt.-% sodium chloride, based on the weight of the rinsing fluid.

According to another aspect of the invention, or expressed in another way, there is also provided a surgical irrigation fluid composition for use as an irrigation fluid for surgery in a human or animal body. The surgical irrigation fluid composition is defined in the same way as the irrigation fluid composition as described above.

Alternatively or as still another aspect of the invention, the surgical irrigation fluid composition is for use as a monitoring fluid in a diagnostic method for the determination of the amount of irrigation fluid, which is absorbed during surgery in a human or animal body. Also in this respect the implementations described above are applicable.

The surgical irrigation fluid composition as described above can be used as the fluid composition in the methods of the first two aspects of the invention.

In a yet further aspect, there is disclosed a ready-to-use kit comprising the surgical irrigation fluid composition as described above. Preferably, the ready-to-use kit further comprises one or more of an instruction manual, a carbohydrate compound measuring device, for example a glucose measuring device, a data storage device, a carbohydrate compound calibrating system, for example a glucose calibrating system, or other auxiliary materials such as a data display device or data storage means.

Without wishing to be bound by theory, the methods, surgical irrigation fluid compositions and ready-to-use kits as described herein can be used to trace or quantify the fluid absorption in a human or animal body with a high accuracy as well as a good reproducibility. For example, the methods, aqueous irrigation fluids and kits can be used in combination with endoscopic surgery of a human or animal subject, in particular transurethral resection surgery of the prostate, wherein the absorption of the irrigation fluid can be quantified, thereby allowing for determining whether or not the patient bears an enhanced risk of suffering from TUR syndrome. The methods can easily be carried out in routine experiments and in standardized forms in clinical laboratories at low costs to the medicinal facilities. Moreover, the methods cannot only be used in combination with monopolar electrocautery, but also in combination with bipolar electrocautery, because the serum sodium concentration of the patients need not be determined.

A further aspect of the invention is the use of glucose for the determination of the amount of irrigation fluid, which is absorbed during surgery in a human or animal body.

Since, as described above, the detection of glucose in the body fluid of a human or animal body can be made by a glucose detector device known in the art, an aspect of the invention is also represented by the use of a glucose detector device or glucose monitoring device for monitoring the amount of surgical irrigation fluid composition absorbed by a human or animal body in surgery, wherein the surgical irrigation fluid composition is as described above.

A final aspect of the invention is represented by the use of the surgical irrigation fluid composition as described above for the manufacture of a medicament for monitoring the amount of surgical irrigation fluid composition absorbed by a human or animal body in surgery, preferably by monitoring at least one body fluid taken from the human or animal body by means of a glucose detector to detect and/or quantify the concentration of glucose therein.

In connection with the last two aspects of the invention, the glucose monitoring device or glucose detector device can be selected among previously known glucose detectors used for other experiments or easily modified therefrom.

The surgery, as used in the different aspects of the invention, is preferably endoscopic surgery, more preferably endoscopic surgery in the genitourinary tract. For example, the endoscopic surgery can be transurethral resection (TUR) or transcervical resection (TCR) surgery. In a particularly preferred implementation, the surgery is transurethral resection of the prostate (TURP) or transcervical resection of the endometrium (TCRE). For example, the surgery may be monopolar or bipolar TURP or may be monopolar or bipolar TRCE.

Preferably, the invention is employed in arthroscopy in particular shoulder arthroscopy, percutaneous lithotripsy or hysteroscopy.

The invention will now be explained in more detail, with reference to the examples and the accompanying figures. The examples and figures should be construed merely as exemplification of preferred embodiments of the invention and not as limiting the scope of the invention in any way.

**Description of the figures**

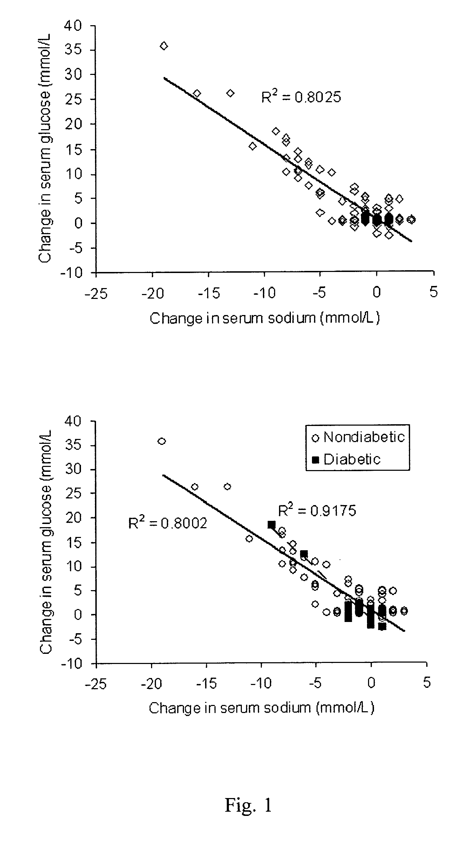

Fig. 1 depicts the change in serum glucose *versus* serum sodium during transurethral resection of the prostate (TURP) using an irrigation fluid containing 5% glucose. A decrease in serum sodium concentration indicates fluid absorption. *Left panel:* all patients. *Right panel:* diabetic patients separated from the non-diabetics.

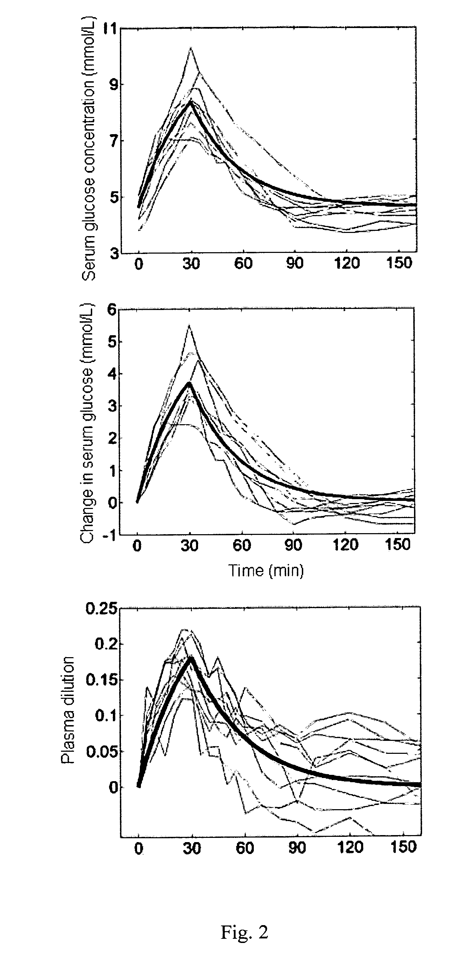

Fig. 2 depicts the serum glucose (*left*), the change in serum glucose (*middle*) and the plasma dilution (*right*) over time for 10 healthy volunteers during a 30-min infusion of 20 mL/kg of acetated Ringer's solution with 1% of glucose. Thin lines represent each subject and the thick line represents the modeled curve based on the mean of the best estimates of the kinetic parameters as shown in Table 2.

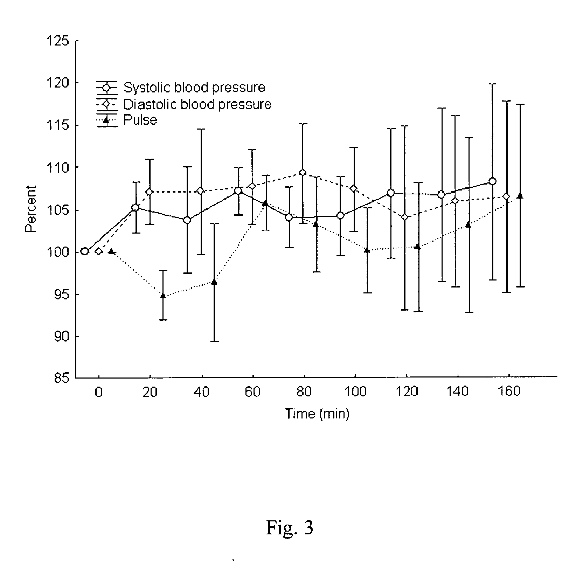

Fig. 3 depicts the mean (SD) of the change in hemodynamics from baseline during and after the 30-min intravenous infusions of acetated Ringer's solution with 1% glucose.

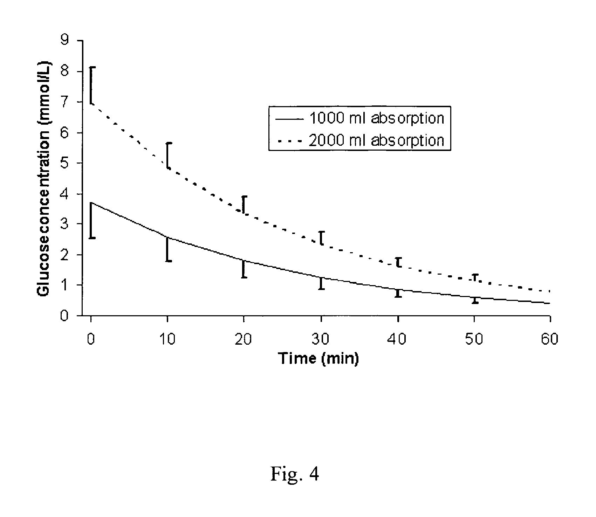

Fig. 4 depicts the mean (SD) of the change in serum glucose concentration over time as predicted by computer simulations. Every possible pattern of absorption of a fluid containing 1% glucose was entered in steps of 250 mL up to 2 L over a 60-min period. The continuous and dotted lines indicate 1,000 and 2,000 mL of absorption, respectively.

**Examples**

**Example 1: clinical study**

In Example 1, it was investigated whether changes in serum glucose concentration provide the same information as the established marker serum sodium during TURP. Monopolar TURP was performed on 250 patients aged between 48 to 96 (mean 74) years and with a body weight of 55 to 135 (mean 79) kg at Southmead & Torbay Hospitals in the United Kingdom. The fasting patients consented to be randomized to receive either 1.5% glycine or 5% glucose in water as the irrigation fluid. Diabetics were not excluded. Blood samples were taken just before and 5 min after the end of each operation for measurement of the serum glucose and sodium concentrations, which were performed by the in-hospital routine clinical chemistry laboratory.

There was a statistically significant linear relationship between the decrease in serum sodium concentration and the increase in serum glucose when 5% glucose in water was used as the irrigation solution (Fig. 1, left). For non-diabetic and diabetic patients, respectively, the coefficients of determination were 0.80 ($P<0.0001$) and 0.92 ($P<0.0001$) (Fig. 1, right).

Those patients who received 1.5% glycine, but did not absorb irrigation fluid, as evidenced by a change in serum sodium by no more than 1 mmol/L, had no significant change in serum glucose during monopolar TURP (mean 0.05 mmol/L, SD 0.65).

Plasma glucose almost doubled during the experimental infusions, and reached a maximum of 8.27 mmol/L (SD 0.95), while the plasma was diluted by 17.7 (3.2) percent (Fig. 2).

The excreted urine at the end of the experiments (160 min) had a volume of 1.14 (0.77) L, which represented 66 (37) % of the infused fluid volume. Mean (SD) for the serum concentrations of sodium and potassium at baseline, at end of the infusion, and at the end of the experiment and the total urinary excretion of sodium and potassium are shown in Table 1. Also shown in the right column of Table 1 is the approximate time from the end of the infusion until levels returned to baseline.

Table 1

|  | Baseline | End infusion | End experiment | Total excretion | Baseline return (min) |
|---|---|---|---|---|---|
| Sodium (mmol/L) | 140.7 | 138.8 | 140.3 | 89.4 | 30 |
| SD Sodium | 1.5 | 2.0 | 2.0 | 35.4 | NA |
| Potassium (mmol/L) | 3.87 | 3.99 | 4.05 | 22.8 | NA |
| SD Potassium | 0.22 | 0.22 | 0.21 | 10.4 | NA |

Glucosuria was not observed in any experiment. The hemodynamic parameters changed mainly at the beginning of the infusion, averaging +9% (systolic pressure), +12% (diastolic pressure) and -7% (heart rate) (Fig. 3).

No adverse reactions were seen during the experimental infusions.

**Example 2: experimental studies**

In Example 2, experimental studies were performed on healthy volunteers and aimed to create a nomogram for the correlation between the irrigation fluid volume and the increase in serum glucose concentration. The glucose was added to acetated Ringer's solution instead of 0.9 % NaCl since the composition of Ringer's is more similar to the extracellular fluid volume.

The studies comprised 10 healthy male volunteers, aged 20 to 41 (mean 30) years and with a body weight of 72 to 111 (mean, 85) kg. After institutional approval by the Ethics Committee of Human Research in Stockholm, the volunteers were enrolled in the study after receiving informed written consent. All experiments were performed at the Clinical Research Center at Södertälje Hospital in Sweden.

After a light breakfast at home, the volunteers were not allowed to eat or drink during the experiment. Prior to study initiation, the volunteers rested supine for 20 min to reach a hemodynamic steady state. A cannula was introduced into the antecubital vein of each arm for infusion and drawing of blood samples, respectively. Acetated Ringer's solution, 20 mL/kg, to which 34.5 ml of glucose 300 mg/mL had been added to reach a concentration of 10 mg/mL (1%), was infused at a constant rate over 30 min. The rationale for lowering the concentration to 1% was that glucose 5%, as indicated by the results of the clinical study, could raise serum glucose to levels far above those that result in glucosuria, and thus seemed unnecessarily high.

Blood samples were collected at 5, 10 and 20-min intervals between 0 and 60 min, 60 and 100 min, and 100 and 160 min, respectively. Analyses [serum glucose concentration, hemoglobin (Hgb), red blood cell count (RBC), mean corpuscular cell volume (MCV) and baseline hematocrit (Hct)] were performed by the hospital clinical chemistry laboratory. Additionally, plasma concentrations of sodium and potassium were analyzed at 0, 30, 60 and 160 minutes. Urine was collected during and immediately after the experiments and analyzed for the content of sodium, potassium, and glucose.

Monitoring comprised non-invasive blood pressure and heart rate using an Omron HEM 722 C1-1 (Omron, Hamburg, Germany).

## Example 3: kinetic analysis and simulations

The kinetics of the glucose infusion was studied to characterize how effectively the volunteers handled the glucose molecules as well as the accompanying water volume. Hence, the pharmacokinetics was analyzed using a one-compartment turnover model, as described in F. Sjöstrand and R. G. Hahn, Br. J. Anaesth. 2003, 90, 600-607, in which the plasma concentration of glucose above baseline (C) at any time (t) resulting from the infusion rate $ki$ is calculated by using the following differential equation:

$$\text{Eqn. 1} \qquad \frac{dC}{dt} = \frac{k_i - (CL \times C(t))}{V_d}$$

in which $V_d$ is the volume of distribution and $CL$ is the glucose clearance.

The best estimates and the associated standard deviation for the unknown parameters in this model, being $V_d$ and $CL$ for exogenous glucose, were estimated for each of the 10 experiments individually by applying a nonlinear least-squares regression routine based on a Gauss-Newton method to the analytical solutions of Eqn. 1 until no parameter changed by more than 0.0001 (0.01%) in each iteration (*cf.* F. Sjöstrand and R. G. Hahn, Br. J. Anaest. 2003, 90, 600-607; R. G. Hahn, J. Anaest. 1997, 78, 144-148; C.-A. Ewaldsson, Anesthesiology 2005, 103, 460-469).

Prediction of the plasma glucose concentration over time for various theoretical infusion regimens were obtained by inserting the best estimates of $V_d$ and $CL$ for the group into the mathematical solution to the Eqn. 1, using the same computer program (Matlab version 4.2, Math Works Inc., Notich, MA) as the one used for analysis. Simulations included 60-min plots of early (first 30 min), late (last 30 min) and continuous absorption patterns. Volumes of up to 2000 mL were tested in steps of 250 mL for every possible combination of early, late and continuous absorption. A nomogram was constructed based on the observation that the post-simulation glucose level decreased approximately 30% per 10 minutes.

The data were normally distributed and thus results were expressed as the mean and standard deviation (SD). Correlations were evaluated by linear regression analysis. $P < 0.05$ was considered statistically significant.

The kinetic analyses yielded that $V_d$ was 15.52 L (SD 2.15) and $CL$ was 0.56 L/min (0.15) for exogenous glucose. The expandable body fluid space was 6.03 L (2.70) with a $k_r$ of 0.19 L/min (0.12) for the infused water volume. Table 2 shows the best estimate and SD for the volume of distribution ($V_d$) and clearance ($CL$) of exogenous glucose (top) and the expandable body fluid space ($V$) and clearance constant ($k_r$) of the infused water volume (bottom) for each subject receiving a 30-min infusion of 20 mL/kg of acetated Ringer's solution with 1% glucose.

Table 2

| | | Subject 1 | Subject 2 | Subject 3 | Subject 4 | Subject 5 |
|---|---|---|---|---|---|---|
| Glucose | $V_d$ (L) | 16.70 | 14.59 | 15.28 | 14.96 | 11.94 |
| | SD | 0.73 | 0.83 | 1.91 | 1.89 | 0.90 |
| | CL (L/min) | 0.42 | 0.59 | 0.66 | 0.78 | 0.50 |
| | SD | 0.02 | 0.03 | 0.06 | 0.07 | 0.03 |
| Water volume | V (L) | 6.32 | 4.19 | 3.79 | 2.92 | 4.38 |
| | SD | 0.47 | 1.22 | 0.35 | 0.50 | 0.40 |
| | $K_r$ (L/min) | 0,21 | 0,44 | 0,20 | 0,35 | 0,100 |
| | SD | 0.01 | 0.06 | 0.01 | 0.03 | 0.01 |
| Body weight | (kg) | 72.0 | 111.0 | 78.0 | 80.0 | 72.0 |

| | | Subject 6 | Subject 7 | Subject 8 | Subject 9 | Subject 10 | Mean | Group SD |
|---|---|---|---|---|---|---|---|---|
| Glucose | $V_d$ (L) | 18.49 | 15.98 | 14.92 | 18.99 | 13.37 | 15.52 | 2.15 |
| | SD | 1.25 | 0.87 | 1.36 | 1.79 | 1.07 | 1.26 | 0.46 |
| | CL (L/min) | 0.48 | 0.44 | 0.38 | 0.82 | 0.52 | 0.56 | 0.15 |
| | SD | 0.03 | 0.02 | 0.03 | 0.06 | 0.03 | 0.04 | 0.02 |
| Water volume | V (L) | 11.57 | 8.06 | 6.19 | 8.61 | 4.26 | 6.03 | 2.70 |
| | SD | 1.16 | 0.69 | 0.70 | 1.22 | 0.48 | 0.72 | 0.35 |
| | $K_r$ (L/min) | 0,12 | 0,20 | 0,11 | 0,10 | 0,12 | 0.19 | 0.12 |
| | SD | 0.02 | 0.02 | 0.01 | 0.02 | 0.01 | 0.02 | 0.02 |
| Body weight | (kg) | 81.0 | 111.0 | 85.0 | 89.0 | 72.0 | 85.1 | 14.8 |

A nomogram was successfully constructed and showed that, regardless of the simulated pattern of fluid absorption over one hour, the uptake of 2 L of irrigation fluid containing glucose 1 % would have increased serum glucose by 6.9 mmol/L (1.7) at the end of surgery (Fig. 4). The corresponding increase for 1 L of irrigation fluid was 3.7 mmol/L (1.6).

**Claims**

1. Method to monitor the fluid absorption of a human or animal body, comprising the steps of:

   (a) contacting a human or animal body with a fluid composition comprising at least one marker compound,
   (b) taking a body fluid sample from the human or animal body,
   (c) measuring the concentration of the marker compound or one of its metabolites in the body fluid sample, and
   (d) calculating the fluid absorption of the body from the concentration of the marker compound or one of its metabolites in the body fluid sample,

   wherein the marker compound is a physiologically acceptable carbohydrate compound.

2. Method for obtaining a set of data of a human or animal body, comprising the steps of:

   (a) bringing into contact a human or animal body with a fluid composition comprising at least one marker compound,
   (b) taking a body fluid sample from the human or animal body,
   (c) measuring the concentration of the marker compound or one of its metabolites in the body fluid sample,
   (d) compiling a set of data from the concentration of the marker compound or one of its metabolites in the body fluid sample,

   wherein the marker compound is a physiologically acceptable carbohydrate compound.

3. Method according to claim 1 or 2, wherein in the step (d) the fluid absorption of the body is calculated or the set of data is compiled as a function of time.

4. Surgical irrigation fluid composition comprising a rinsing fluid and a marker compound, wherein the rinsing fluid comprises sterile water and the marker compound is a physiologically acceptable carbohydrate compound in an amount detectable via a human or animal body, which is subject to surgery, wherein the physiologically acceptable carbohydrate compound is preferably glucose.

5. Surgical irrigation fluid composition according to claim 4, further comprising a haemolysis preventing solute, preferably selected from glycine, mannitol and sorbitol and mixtures thereof, wherein the amount of the haemolysis preventing solute preferably is within the range of 0.1 to 5.0 wt.-%, more preferably 0.5 to 2.5 wt.-%, even more preferably 1.0 to 2.0 wt.-%, based on the weight of the rinsing fluid.

6. Surgical irrigation fluid composition according to claims 4 or 5, wherein the rinsing fluid comprises Ringer's acetate solution.

7. Surgical irrigation fluid composition according to any one of claims 4 to 6 for use as an irrigation fluid for surgery in a human or animal body.

8. Surgical irrigation fluid composition according to any one of claims 4 to 6 for use as as a monitoring fluid in a diagnostic method for the determination of the amount of irrigation fluid, which is absorbed during surgery in a human or animal body.

9. Surgical irrigation fluid composition according to any one of claims 4 to 8, wherein the surgery is endoscopic surgery, preferably surgery in the genitourinary tract.

10. Surgical irrigation fluid composition according to claim 9, wherein the endoscopic surgery is transurethral resection (TUR) or transcervical resection surgery (TCR), preferably transurethral resection of the prostate (TURP).

11. Ready-to-use kit comprising the surgical irrigation fluid composition according to any one of claims 4 to 10.

12. Use of a surgical irrigation fluid composition according to any one of claims 4 to 10 for the manufacture of a medicament for monitoring the amount of irrigation fluid absorbed by a human or animal body.

13. Use of a glucose detector as a device for monitoring the amount of surgical irrigation fluid composition according to any one of claims 4 to 10, which is absorbed by a human or animal body.

14. Method according to any one of claims 1 to 3, wherein the surgical irrigation fluid composition according to any one of claims 4 to 10 is used as the fluid composition.

15. Use of the method, surgical irrigation fluid or ready-to-use kit, according to any of the preceding claims, for arthroscopy, shoulder arthroscopy, percutaneous lithotripsy or hysteroscopy.

Fig. 1

Fig. 2

Fig. 3

Fig. 4

Europäisches
Patentamt

European
Patent Office

Office européen
des brevets

**EUROPEAN SEARCH REPORT**

Application Number

EP 09 01 1667

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GEHRING H ET AL: "Detecting and quantifying absorbed irrigation fluid by measuring mannitol and sorbitol concentrations in serum samples, and by ethanol monitoring" BJU INTERNATIONAL, vol. 89, no. 3, February 2002 (2002-02), pages 202-207, XP002567653 ISSN: 1464-4096 * the whole document * * figures 2,3 * | 1,3,14 | INV. G01N33/50 A61K31/19 |
| X | HAHN R G: "Irrigating fluids in endoscopic surgery" BRITISH JOURNAL OF UROLOGY, vol. 79, no. 5, 1997, pages 669-680, XP002567656 ISSN: 0007-1331 * the whole document * * page 669 - column 1, paragraph 4 * * figure 4 * | 1,3,14 | |
| X | NABER K ET AL: "Measurement of absorption of irrigating fluids during transurethral resection of the prostate and its clinical importance" UROLOGE - AUSGABE A 1971, vol. 10, no. 6, 1971, pages 261-265, XP008118509 ISSN: 0340-2592 * the whole document * * abstract * * figure 2 * | 1,3,14 | TECHNICAL FIELDS SEARCHED (IPC) G01N A61K |

-/--

~~The present search report has been drawn up for all claims~~

| Place of search | Date of completion of the search | Examiner |
|---|---|---|
| The Hague | 12 February 2010 | Jenkins, Gareth |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 09 01 1667

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DAVIS J M ET AL: "FLUID AVAILABILITY OF SPORTS DRINKS DIFFERING IN CARBOHYDRATE TYPE AND CONCENTRATION" AMERICAN JOURNAL OF CLINICAL NUTRITION, vol. 51, no. 6, 1990, pages 1054-1057, XP007911559 ISSN: 0002-9165 * the whole document * * figures 1,2 * | 1,3,14 | |
| A | US 2006/073098 A1 (WANG YANMING [US]) 6 April 2006 (2006-04-06) * the whole document * * claims 1,2,10 * | 1,3,14 | |
| A | DATABASE BIOSIS [Online] BIOSCIENCES INFORMATION SERVICE, PHILADELPHIA, PA, US; 1993, SANDSTROM KERSTIN ET AL: "Metabolic consequences of different perioperative fluid therapies in the neonatal period" XP002567655 Database accession no. PREV199395123873 * abstract * & ACTA ANAESTHESIOLOGICA SCANDINAVICA, vol. 37, no. 2, 1993, pages 170-175, ISSN: 0001-5172 | 1,3,14 | TECHNICAL FIELDS SEARCHED (IPC) |
| T | PIROS DAVID ET AL: "Glucose as a Marker of Fluid Absorption in Bipolar Transurethral Surgery" ANESTHESIA & ANALGESIA, vol. 109, no. 6, December 2009 (2009-12), pages 1850-1855, XP008118468 ISSN: 0003-2999 * the whole document * | 1,3,14 | |

The present search report has been drawn up for all claims

| Place of search | Date of completion of the search | Examiner |
|---|---|---|
| The Hague | 12 February 2010 | Jenkins, Gareth |

EPO FORM 1503 03.82 (P04C01)

**Europäisches Patentamt**
**European Patent Office**
**Office européen des brevets**

---

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

---

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1(completely); 3, 14(partially)

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION
SHEET B**

Application Number

EP 09 01 1667

The Search Division considers that the present European patent application does not comply with the requirements of unity of invention and relates to several inventions or groups of inventions, namely:

```
1. claims: 1(completely); 3, 14(partially)

            Method of claim 1.
                 ---

2. claims: 2(completely); 3, 14(partially)

            Method of claim 2.
                 ---

3. claims: 4-11(completely); 14(partially)

            Product of claim 4.
                 ---

4. claim: 12

               Use according to claim 12.
                    ---

5. claim: 13

               Use according to claim 13.
                    ---

6. claim: 15

               Use according to claim 15.
                    ---
```

**ANNEX TO THE EUROPEAN SEARCH REPORT
ON EUROPEAN PATENT APPLICATION NO.**

EP 09 01 1667

This annex lists the patent family members relating to the patent documents cited in the above-mentioned European search report.
The members are as contained in the European Patent Office EDP file on
The European Patent Office is in no way liable for these particulars which are merely given for the purpose of information.

12-02-2010

| Patent document cited in search report | Publication date | Patent family member(s) | Publication date |
|---|---|---|---|
| US 2006073098 A1 | 06-04-2006 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **F. Sjöstrand ; R. G. Hahn.** *Br. J. Anaesth.,* 2003, vol. 90, 600-607
- **F. Sjöstrand ; R. G. Hahn.** *Br. J. Anaest.,* 2003, vol. 90, 600-607
- **R. G. Hahn.** *J. Anaest.,* 1997, vol. 78, 144-148
- **C.-A. Ewaldsson.** *Anesthesiology,* 2005, vol. 103, 460-469